# EUROPEAN PATENT SPECIFICATION

(11) **EP 1 891 098 B1**
(45) Date of publication and mention of the grant of the patent: **24.08.2011**
(21) Application number: 06767209.7
(22) Date of filing: 16.06.2006
(51) Int. Cl.: C07K 14/395, C12N 15/81, C12P 11/00, C12N 1/18, C12G 1/022

(54) **SULFATE ION TRANSPORTER GENE AND USE THEREOF**
SULFATIONEN-TRANSPORTER-GEN UND VERWENDUNG DAVON
GÈNE TRANSPORTEUR DE L'ION SULFATE ET SON UTILISATION

(30) Priority: 17.06.2005 JP 2005177821
(43) Date of publication of application: 27.02.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 6188503 (JP); KODAMA, Yukiko, Mishima-gun, Osaka 6188503 (JP); SHIMONAGA, Tomoko, Mishima-gun, Osaka 6188503 (JP)
(74) Representative: Vossius & Partner
(86) International application number: PCT/JP2006/312559
(87) International publication number: WO 2006/135110

(56) References cited:
- DE-A1- 10 116 101
- US-A1- 2004 265 862
- CHEREST HELENE ET AL: "Molecular characterization of two high affinity sulfate transporters in Saccharomyces cerevisiae" GENETICS, vol. 145, no. 3, 1997, pages 627-635, XP002403734 ISSN: 0016-6731 cited in the application
- DATABASE EMBL [Online] 12 April 2001 (2001-04-12), "Saccharomyces pastorianus sulfate transporter SUL2-LA gene, partial cds." XP002403765 retrieved from EBI accession no. EM_PRO:AF364410 Database accession no. AF364410
- DATABASE EMBL [Online] 10 October 2004 (2004-10-10), "Sequence 222 from patent US 6723837." XP002403766 retrieved from EBI accession no. EM_PRO:AR527919 Database accession no. AR527919
- HANSEN JORGEN ET AL: "Inactivation of MET10 in brewer's yeast specifically increases SO-2 formation during beer production" NATURE BIOTECHNOLOGY, vol. 14, no. 11, 1996, pages 1587-1591, XP008070294 ISSN: 1087-0156 cited in the application
- JAMES A B ET AL: "The molecular genetics of sulphate transport in brewer's yeast" JOURNAL OF THE INSTITUTE OF BREWING, vol. 105, no. 1, January 1999 (1999-01), page 9, XP008070266 & 2ND TECHNICAL MEETING OF THE EUROPEAN BREWERY CONVENTION BREWING SCIENCE GROUP; EDINBURGH, SCOTLAND, UK; SEPTEMBER 6-9, 1998 ISSN: 0046-9750

## Description

### TECHNICAL FIELD

The present invention relates to a sulfate ion transporter gene and use thereof, in particular, a brewery yeast for producing alcoholic beverages with enhanced flavor stability, and a method for producing said beverages. More particularly, the present invention relates to a yeast, whose sulfite-producing capability that contribute to a product's flavor, is adjusted by controlling expression level of SUL2 gene encoding brewery yeast sulfate ion transporter Sul2p, in particular the non-ScSUL2 gene specific to a lager brewing yeast, and to a method for producing alcoholic beverages with said yeast

### BACKGROUND ART

Sulfite has been known as a compound having high anti-oxidative activity, and thus has been widely used in the fields of food, beverages, pharmaceutical products or the like (for example, Japanese Laid-Open Patent Application Nos. H06-040907 and 2000-093096). In alcoholic beverages, sulfate has been used as an anti-oxidant For example, in view of an important role in quality maintenance for wine that needs long time aging, addition of up to 350 ppm (parts per million) of residual concentration is permitted by the Ministry of Health, Welfare and Labor in Japan. Further, it is also known that shelf life (quality maintained period) varies depending upon sulfite concentration of a product in beer brewing. Thus, it is quite important to increase the content of this compound from the viewpoint of flavor stability or the like.

The easiest way to increase the sulfite content in a product is to add sulfite. However, sulfite is treated as a food additive, resulting in some problems such as constraint of product development and the food additive related negative image of consumers.

In the meanwhile, yeast produces, by biosynthesis, sulfur containing compounds required for yeast life cycle. Sulfite is produced as an intermediate metabolite. Thus, with use of the capability of yeasts, sulfite content in a product can be increased without addition of sulfite.

Methods of increasing sulfite content in a fermentation liquor during brewing process Include (1) a method based on process control, and (2) a method based on breeding of yeast. In the method based on a process control, since the amount of sulfate produced is in inverse proportion to the amount of initial oxygen supply, amount of oxygen to be supplied can be reduced to increase amount of sulfite produced and to prevent oxidation.

On the other hand, gene manipulation techniques are used in the method based on breeding of yeast. In sulfur metabolism of yeasts, sulfite is an intermediate produced in biosynthesis of sulfur-containing amino acids or sulfur-containing vitamins. Sulfite is produced by reduction of three step reactions of sulfate ions taken up from outside of cells.

The MET3 gene is a gene encoding an enzyme that catalyzes a first reaction; the MET14 gene is a gene encoding an enzyme that catalyzes a second reaction. Korch et al. attempted to increase a sulfite-producing capability of yeasts by increasing the expression level of these two genes, and found that MET14 is more effective (C. Korch et al., Proc. Eur. Brew. Conv. Conger., Lisbon, 201-208, 1991). Hansen et al. attempted to increase production amount of sulfite by disrupting a MET10 gene encoding a reductase for sulfite to prevent reduction of sulfite produced (J. Hansen et al., Nature Biotech.,1587-1591,1996).

Further, Fujimura et al. attempted to increase sulfite content in beer by increasing expression level of a non-ScSSU1 gene unique to a lager brewing yeast among SSU1 genes encoding sulfite ion afflux pump of yeast to promote excretion of sulfite to outside the fungal body (Fujimura et al., Abstract of 2003 Annual Conference of the Japan Society for Bioscience, Biotechnology and Agrochem., 159, 2003).

### DISCLOSURE OF INVENTION

Nevertheless, new methods and materials are needed for increasing the yeast-produced amount of sulfite to improve the shelf-life and flavor stability of the alcoholic beverage produced by the yeast. As mentioned above, the easiest way to increase sulfite content in a product is addition of extraneous or non-yeast produced sulfite. However, it is desirable to minimize use of food additives in view of recent consumers' preference, i.e., avoidance of food additives and use of natural materials. Thus, it is desirable to achieve sulfite content effective for flavor stability without adding sulfite from outside. However, the method based on a process control as described above may not be practical since shortage of oxygen may cause decrease in growth rate, resulting in delay in fermentation and quality loss.

Further, in breeding of yeast using gene manipulation techniques, genes involved in reduction reaction of sulfate ions to sulfite ions have been main targets. Among such targets, there is a report stating that ten times or more sulfite content was achieved (J. Hansen et al., Nature Biotech., 1587-1591, 1996). However, there are problems such as delay in fermentation and increase of undesirable flavor ingredients such as acetaldehyde and 1-propanol. Thus, the yeast is not good for practical use. Thus, there has been a need for a method for breeding yeast capable of producing abundant amount of sulfate without impairing the fermentation rates and quality of the products.

The materials and methods disclosed herein solve the above problems, and as a result succeeded in identifying and isolating a gene encoding sulfate ion transporter from lager brewing yeast which has advantageous effects in comparison to the existing proteins. Moreover, a yeast was transformed by introducing and expressing the obtained gene to confirm that the amount of sulfite produced was increased, thereby completing the present invention.

Thus, the present invention relates to a novel sulfate ion transporter gene existing specifically in a lager brewing yeast, to a protein encoded by said gene, to a transformed yeast in which the expression of said gene is controlled, to a method for controlling the amount of sulfite in a product by using a yeast in which the expression of said gene is controlled. More specifically, the present invention provides the following polynucleotides, a vector comprising said polynucleotide, a transformed yeast into which said vector has been introduced, a method for producing alcoholic beverages by using said transformed yeast, and the like.
(1) A polynucleotide selected from the group consisting of
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2;
   (c) a polynucleotide comprising a polynucleotide encoding a protein consisting of the amino acid sequence of SEQ ID NO:2 with 1 to 50 amino acids thereof being deleted, substituted, inserted and/or added, and having a sulfate ion transporter activity; and
   (d) a polynucleotide comprising a polynucleotide encoding a protein having an amino acid sequence having 94% or higher identity with the amino acid sequence of SEQ ID NO:2, and having a sulfate ion transporter activity.
(2) The polynucleotide of (1) above consisting of SEQ ID NO: 1.
(3) The polynucleotide of (1) above encoding a protein of SEQ ID NO: 2.
(4) The polynucleotide of (1) above, wherein the polynucleotide is DNA.
(5) A polynucleotide selected from the following polynucleotides (j) to (m):
   (j) a polynucleotide encoding RNA of a nucleotide sequence complementary to a transcript of the polynucleotide (DNA) according to [4] above;
   (k) a polynucleotide encoding RNA that represses the expression of the polynucleotide (DNA) according to [4] above through RNAi effect;
   (1) a polynucleotide encoding RNA having an activity of specifically cleaving a transcript of the polynucleotide (DNA) according to [4] above; and
   (m) a polynucleotide encoding RNA that represses expression of the polynucleotide (DNA) according to [4] above through co-supression effect.
(6) A protein encoded by the polynucleotide of any of (1) to (4) above.
(7) A vector comprising the polynucleotide of any of (1) to (4) above.
(8) The vector of (7) above, which comprises the expression cassette comprising the following components:
   (a) a promoter that can be transcribed in a yeast cell;
   (b) any of the polynucleotides described in (1) to (4) above linked to the promoter in a sense or antisense direction; and
   (c) a signal that can function in a yeast with respect to transcription termination and polyadenylation of a RNA molecule.
(9) A yeast, wherein the vector of (7) above is introduced.
(10) The yeast of (9) above, wherein sulfite producing ability is enhanced by introducing the vector of (7) above.
(11) A yeast, Wherein an expression of the polynucleotide (DNA) of (4) above is repressed by introducing the vector of (8) above, or by disrupting a gene related to the polynucleotide (DNA) of (4) above.
(12) The yeast of (9) above, wherein a sulfite-producing ability is elevated by increasing an expression level of the protein of (6) above.
(13) A method for producing an alcoholic liquor comprising culturing the yeast of any one of (9) through (12) above under conditions for producing an alcoholic beverage.
(14) The method for producing an alcoholic liquor of (13) above, wherein the brew is a malt liquor.
(15) The method for producing an alcoholic liquor of (13) above, wherein the brew is a wine.
(16) A method for assessing a test yeast for its sulfite-producing ability, comprising using a primer or a probe designed based on a nucleotide sequence of a sulfate ion transporter gene having the nucleotide sequence of SEQ ID NO: 1.
   Also disclosed is a method for selecting a yeast having a high sulfirous acid-producing ability by using the method in (16) above and a method for producing an alcoholic liquor (for example, beer) by using the yeast selected with such a method.
(17) A method for assessing a test yeast for its sulfite-producing capability, comprising: culturing a test yeast; and measuring an expression level of a sulfate ion transporter gene having the nucleotide sequence of SEQ ID NO: 1.
   Also disclosed is a method for selecting a yeast having a high sulforous acid producing ability, which comprises assessing a test yeast by the method described in (17) above and selecting a yeast having a high expression level of sulfur ion transporter gene as well as a method for producing an alcoholic liquor (for example, beer) by using the yeast selected with such a method.
(18) A method for selecting a yeast, comprising: culturing test yeasts; quantifying the protein of (6) above or measuring an expression level of a sulfate ion transporter gene having the nucleotide sequence of SEQ ID NO: 1; and selecting a test yeast having said protein amount or said gene expression level according to a target capability of producing sulfite.
(19) The method for selecting a yeast of (18) above, comprising: culturing a reference yeast and test yeasts; measuring an expression level of a sulfate ion transporter gene having the nucleotide sequence of SEQ ID NO: 1 in each yeast; and selecting a test yeast having the gene expressed higher or lower than that in the reference yeast.
(20) The method for selecting a yeast of (18) above comprising: culturing a reference yeast and test yeasts; quantifying the protein of (6) above in each yeast; and selecting a test yeast having said protein for a larger or smaller amount than that in the reference yeast.
(21) A method for producing an alcoholic beverage comprising: conducting fermentation for producing an alcoholic beverage using the yeast according to any one of (9) to (12) or a yeast selected by the method according to any one of (18) to (20); and adjusting the production amount of sulfite.

According to the method for producing alcoholic beverages by using a yeast transformed with a sulfite ion transporter polynucleotide operably linked to a vector, the content of sulfite having an anti-oxidative activity in a product can be increased so that alcoholic beverages can be produced with enhanced flavor and improved shelf life.

### BRIEF DESCRIPTION OF DRAWINGS

Figure 1 shows the cell growth with time upon beer brewing testing using the SUL2 gene-highly expressed strains. The horizontal axis represents fermentation time while the vertical axis represents optical density at 660 nm (OD660).
Figure 2 shows the sugar consumption with time upon beer brewing testing using the SUL2 gene-highly expressed strains. The horizontal axis represents fermentation time while the vertical axis represents apparent extract concentration (w/w%).
Figure 3 shows the sulfite concentration in finished beer.

### BEST MODES FOR CARRYING OUT THE INVENTION

In the known method of increasing expression level of a sulfite ion afflux pump, suitable fermentation rate can be maintained since superfluous sulfate is not accumulated in a fungal body. However, there is a possibility that taking up of sulfate ion as substrate can be a limiting factor. Thus, disclosed herein are materials and methods that enhance sulfite production by increasing a sulfate ion-taking up capability of a yeast.

The present inventors have studied based on this conception and as a result, isolated and identified nonSc-ScSUL2 gene encoding a sulfate ion transporter unique to lager brewing yeast based on the lager brewing yeast genome information mapped according to the method disclosed in Japanese Laid-Open Patent Application No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide of the nucleotide sequence of SEQ ID NO:1; and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding a sulfate ion transporter gene derived from lager brewing yeast and may include other polynucleotides encoding proteins having equivalent functions to said protein. Proteins with equivalent functions include, for example, (c) a protein of an amino acid sequence of SEQ ID NO: 2 with 1 to 50 amino acids thereof being deleted, substituted, inserted and/or added and having sulfate ion transporter activity.

Such proteins include a protein consisting of an amino acid sequence of SEQ ID NO: 2 with, for example, 1 to 50, 1 to 40, 1 to 39, 1 to 38,1 to 37,1 to 36,1 to 35,1 to 34, 1 to 33, 1 to 32, 1 to 31, 1 to 30, 1 to 29,1 to 28, 1 to 27, 1 to 26, 1 to 25,1 to 24.1 to 23, 1 to 22,1 to 21, 1 to 20,1 1 to 19,1 to 18,1 to 17,1 to 16,1 to 15,1 to 14,1 to 13,1 to 12,1 to 11,1 to 10,1 to 9,1 to 8,1 to 7,1 to 6 (1 to several amino acids), 1 to 5, 1 to 4, 1 to 3, I to 2, or 1 amino acid residues thereof being deleted, substituted, inserted and/or added and having a sulfate ion transporter activity. In general, the number of deletions, substitutions, insertions, and/or additions is preferably smaller. In addition, such proteins include (d) a protein having an amino acid sequence with 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having a sulfate ion transporter activity. In general, the percentage identity is preferably higher.

Sulfate ion transporter activity may be measured, for example, by a method of Cherest et al. as described in Genetics, 145: 627-635,1997.

Furthermore, the present invention also discloses (e) a polynucleotide comprising a polynucleotide which hybridizes to a polynucleotide consisting of a nucleotide sequence complementary to the nucleotide sequence of SEQ ID NO: 1 under stringent conditions and which encodes a protein having sulfate ion transporter activity; and (f) a polynucleotide comprising a polynucleotide which hybridizes to a polynucleotide complementary to a nucleotide sequence of encoding a protein of SEQ ID NO: 2 under stringent conditions, and which encodes a protein having sulfate ion transporter activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or DNA encoding the amino acid sequence of SEQ ID NO: 2 as a probe. The hybridization method may be a method described, for example, in MOLECULAR CLONING 3rd Ed., CURRENT PROTOCOLS IN MOLECULAR BIOLOGY, John Wiley & Sons 1987-1997.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized DNA.

Polynucleotides that can be hybridized include polynucleotides having 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to DNA encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (Proc. Natl. Acad Sci USA, 87: 2264-2268, 1990; Proc. Natl Acad Sci. USA, 90:5873,1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length =12. When an amino acid sequence is sequenced using BLASTX, the parameters are, for example, score = 50 and word length = 3. When BLAST and Gapped BLAST programs are used, default parameters for each of the programs are employed.

The polynucleotide of the present invention includes (j) a polynucleotide encoding RNA having a nucleotide sequence complementary to a transcript of the polynucleotide (DNA) according to (4) above; (k) a polynucleotide encoding RNA that represses the expression of the polynucleotide (DNA) according to (4) above through RNAi effect; (1) a polynucleotide encoding RNA having an activity of specifically cleaving a transcript of the polynucleotide (DNA) according to (4) above; and (m) a polynucleotide encoding RNA that represses expression of the polynucleotide (DNA) according to (4) above through co-supression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above DNA is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics. 24(2). 180-183, 2000 Feb.; Genesis, 26(4), 240-244,2000 April; Nature, 407:6802, 319-20, 2002 Sep. 21; Genes & Dev., Vol.16, (8), 948-958, 2002 Apr.15; Proc. natl. Acad. Sci. USA., 99(8), 5515-5520, 2002 Apr. 16; Science, 296(5567), 550-553, 2002 Apr. 19; Proc Natl. Acad. Sci. USA, 99:9, 6047-6052, 2002 Apr. 30; Nature Biotechnology, Vol.20 (5), 497-500, 2002 May; Nature Biotechnology, Vol. 20(5), 500-505, 2002 May; Nucleic Acids Res., 30:10, e46,2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; Nucl. Acids. Res. 19: 6751, 1991; Protein Eng 3: 733;1990; Nucl. Acids Res. 19: 3875, 1991; Nucl. Acids Res. 19: 5125, 1991; Biochem Biophys Res Commun 186: 1271, 1992). In addition, the phrase "polynucleotide encoding RNA that represses DNA expression through co-supression effect" refers to a nucleotide that inhibits functions of target DNA by "co-supression".

The term "co-supression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-supression effect can also be found in various publications (see, e.g., Smyth DR: Curr. Biol. 7: R793, 1997, Martienssen R: Curr. Biol. 6: 810, 1996).

### 2. Protein of the present invention

The present invention also provides proteins encoded by any of the polynucleotides (a) to (d) above. A preferred protein of the present invention comprises an amino acid sequence of SEQ ID NO:2 with 1 to 50 amino acids thereof being deleted, substituted, inserted and/or added, and has sulfate ion transporter activity.

Such protein includes those having an amino acid sequence of SEQ ID NO: 2 with amino acid residues thereof of the number mentioned above being deleted, substituted, inserted and/or added and having a sulfate ion transporter activity. In addition, such protein includes those having homology as described above with the amino acid sequence of SEQ ID NO: 2 and having sulfate ion transporter activity.

Such proteins may be obtained by employing site-directed mutation described for example, in MOLECULAR CLONING 3rd Ed., CURRENT PROTOCOLS IN MOLECULAR BIOLOGY, Nuc. Acids. Res., 10: 6487 (1982), Proc. Natl Acad Sci. USA 79: 6409 (1982), Gene 34: 315 (1985), Nuc. Acids. Res., 13: 4431 (1985), Proc. Natl Acad Sci. USA 82: 488 (1985).

Deletion, substitution, insertion and/or addition of 1 to 50 amino acid residues in an amino acid sequence of the protein of the invention means that 1 to 50 amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2.4-diaminobutanoic acid. 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega. PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides described in (a) to (d) above or the polynucleotides described in (j) to (m) above. Generally, the vector of the present invention comprises an expression cassette including as components (a) a promoter that can transcribe in a yeast cell; (b) a polynucleotide described in any of (a) to (d) above that is linked to the promoter in sense or antisense direction; and (c) a signal that functions in the yeast with respect to transcription termination and polyadenylation of RNA molecule. According to the present invention, in order to highly express the protein of the invention described above upon brewing alcoholic beverages (e.g., beer) described below, these polynucleotides are introduced into the promoter in the sense direction to promote expression of the polynucleotide (DNA) described in any of (a) to (d) above. In order to repress the expression of the above protein of the invention upon brewing alcoholic beverages (e.g., beer) as described below, the polynucleotide is introduced into the promoter in the antisense direction to repress the expression of the polynucleotide (DNA) described in any of (a) to (d). In order to repress the above protein of the invention, the polynucleotide may be introduced such that the polynucleotide of any of the (j) to (m) is expressed. According to the present invention, the target gene (DNA) may be disrupted to repress the expression of the DNA or the protein. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951(1979), Methods in Enzymology, 101, 202(1983), Japanese Laid-Open Patent Application No.6-253826).

A vector introduced in the yeast may be any of a multicopy type (YEp type), a single copy type (YCp type), or a chromosome integration type (YIp type). For example, YEp24 (J. R. Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad Sci: US4, 76: 1035, 1979) is known as a YIp type vector, all of which are readily available.

Promoters/terminators for adjusting gene expression in yeast may be in any combination as long as they function in the brewery yeast and they have no influence on the concentration of amino acid, sugar, higher alcohol or ester in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP 1) (Marin et al., Proc. Natl. Acad Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, *Saccharomyces carlsbergensis* NCYC453 or NCYC456, or *Saccharomyces cerevisiae* NBRC1951, NBRC1952, NBRC1953 or NBRC1954 may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as . *Saccharomyces pastorianus* may be used preferably.

A yeast transformation method may be a generally used known method. For example, methods that can be used include but not limited to an electroporation method (Meth Enzym., 194: 182 (1990)), a spheroplast method (Proc. NotL. Acad Sci. USA, 75: 1929(1978)), a lithium acetate method (J. Bacteriology, 153: 163 (1983)), and methods described in Proc. Natl. Acad Sci USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali ion metal ion, preferably lithium ion at a concentration of about 1 to 2 M. After the cell is left to stand at about 30°c for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant

Other general cloning technique may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product This yeast can be used to produce a desired alcoholic beverage with enhanced flavor with an increased content of sulfite. In addition, yeasts to be selected by the yeast assessment method of the present invention can also be used. The target alcoholic beverages include, for example, but not limited to beer, sparkling liquor (*happoushu*) such as a beer-taste beverage, wine, whisky, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with an increased content of sulfite. Thus, according to the present invention, alcoholic beverages with enhanced flavor can be produced using the existing facility without increasing the cost.

Further, since in a yeast wherein said gene is highly expressed, a sulphate ion in the culture medium is efficiently incorporated, well growth of yeast and/or alcoholic fermentation may be possible when a raw material containing low sulfur source, e.g., a wort having low malt ratio in the case of beer.

Alternatively, in a yeast wherein the function of synthetic system for sulfur-containing amino acid is too active, sulfur-containing compounds including hydrogen sulfide as an intermediate-metabolite in the pathway, which cause undesirable off-flavor for alcoholic beverages, are sometimes generated in large amounts and accumulated. By suppressing or disrupting said gene function of such yeast, incorporation of sulphate ion as a starting material may be suppressed. As a result, an alcoholic beverage wherein the off flavor is reduced, can be produced.

### 5. Yeast assessment method of the invention

The present invention relates to a method for assessing a test yeast for its sulfite-producing capability by using a primer or a probe designed based on a nucleotide sequence of a sulfate ion transporter gene having the nucleotide sequence of SEQ ID NO:1. General techniques for such assessment method is known and is described in, for example, WO01/040514, Japanese Laid-Open Patent Application No. 8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the sulfate ion transporter gene, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PER method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of the yeast to produce sulfite as determined by whether the molecular weight of the amplified product is a size that contains the DNA molecule of the specific part In addition, by analyzing the nucleotide sequence of the amplified product, the capability may be predicted and/or assessed more precisely.

Moreover, in the present invention, a test yeast is cultured to measure an expression level of the sulfate ion transporter gene having the nucleotide sequence of SEQ ID NO: 1 to assess the test yeast for its sulfite-producing capability. In this case, the test yeast is cultured and then mRNA or a protein resulting from the sulfate ion transporter gene is quantified. The quantification of mRNA or protein may be carried out by employing a known technique. For example, RNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the sulfate ion transporter gene having the nucleotide sequence of SEQ ID NO: 1 are measured to select a test yeast with the gene expression level according to the target capability of producing sulfite, thereby selecting a yeast favorable for brewing desired alcoholic beverages. In addition, a reference yeast and a test yeast may be cultured so as to measure and compare the expressions level of the gene in each of the yeasts, thereby selecting a favorable test yeast. More specifically, for example, a reference yeast and one or more test yeasts are cultured and an expression level of the sulfate ion transporter gene having the nucleotide sequence of SEQ ID NO:1 is measured in each yeast By selecting a test yeast with the gene expressed higher than that in the reference yeast, a yeast suitable for brewing alcoholic beverages can be selected.

Alternatively, test yeasts are cultured and a yeast with a higher sulfite-producing capability is selected, thereby selecting a yeast suitable for brewing desired alcoholic beverages.

In these cases, the test yeasts or the reference yeast may be, for example, a yeast introduced with the vector of the invention, an artificially mutated yeast or a naturally mutated yeast. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see*, *e.g.*, Yasuji Oshima Ed., BIOCHEMISTRY EXPERIMENTS vol. 39, Yeast Molecular Genetic Experiment, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g.*, *S. pastorianus*, *S. cerevisiae*, and *S. carlsbergensis*). According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954 may be used. Further, whisky yeasts such as *Saccharomyces cerevisiae* NCYC90; wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of SUL2 Gene

Two novel SUL2 genes from a lager brewing yeast genome were found, as a result of a search utilizing the comparison database described in Japanese Laid-Open Patent Application No. 2004-283169. Based on the homology to the nucleotide sequence of the SUL2 gene of *Saccharomyces cerevisiae* disclosed in SGD (*Saccharomyces* genome database; Intemet:<URL:http://www.yeastgenome.org>), the newly found gene having higher homology (99%) is designated as ScSUL2, and one having lower homology (80%) as non-ScSUL2. Based on the acquired nucleotide sequence information, primers were designed to amplify the full-length genes, respectively. PCR was carried out using chromosomal DNA of a genome sequencing strain, *Saccharomyces pastorianus* Weihenhephan 34/70 strain, as a template to obtain DNA fragments (about 2.7 kb each) including either of the full-length gene of ScSUL2 and that of non-ScSUL2. The amplification of the ScSUL2 gene was conducted with primers ScSUL2_for (SEQ ID NO: 3) and ScSUL2_rv (SEQ ID NO: 4). The amplification of the non-ScSUL2 gene was conducted with primers non-ScSUL2_for (SEQ ID NO: 5) and non-ScSUL2_rv (SEQ ID NO: 6).

The thus-obtained nonScSUL2 gene fragment or non-ScSUL2 gene fragment was inserted into pCR2.1-TOPO vector (Invitrogen) by TA cloning. The nucleotide sequences of ScSUL2 and non-ScSUL2 genes were analyzed according to Sanger's method (F. Sanger, Science, 214: 1215, 1981) to confirm the nucleotide sequence. Alter confirming that the nucleotide sequences are correct, the vectors were designated as TOP/ScSUL2 and TOP/non-ScSUL2, respectively.

### Example 2: Constitutive Expression of SUL2 Genes

Each of the plasmids TOPO/ScSUL2 described in Example 1 was digested with restriction enzymes SacI and NotI to prepare a DNA fragment of about 2.7 kb including ScSUL2 gene. This fragment was linked to pUP3GLP2 treated with restriction enzymes SacI and NotI, thereby constructing a ScSUL2 constitutive expression vector, pUP-ScSUL2. Also, following the same procedures, non-ScSUL2 constitutive expression vector was constructed. The yeast expression vector, pUP3GLP2, is a YIp type (chromosome integration type) vector having orotidine-5-phosphoric acid decarboxylase gene URA3 at the homologous recombinant site. The introduced gene was constitutively expressed by the promoter and terminator of glycerylaldehyde-3-phosphoric acid dehydrogenase gene, TDH3. Drug-resistant gene YAP as a selective marker for yeast was introduced under the control of the promoter and terminator of galactokinase GAL1, whereby the expression is induced in a culture media comprising galactose. Ampicillin-resistant gene Amp^{r} as a selective marker for *E. coli* was also included.

The constitutive expression vector prepared by the method above was used to transform *Saccharomyces pastorianus* Weihenstephan 34/70 strain according to the method described in Japanese Laid-Open Patent Application No. 07-303475. Right assessment on the SUL2 gene cannot be conducted if sulfite is accumulated within the fungal body since the yeast itself is damaged by sulfite. Thus, first, a strain in which non-ScSSU1 gene encoding a sulfite efflux pump is highly expressed, was prepared according to the method described in Japanese Patent Application Laid-Open No. 2004-283169, and designated as TFO010.. Then, transformation was conducted to obtain ScSUL2 gene- or non-ScSUL2 gene-highly expressed strain using TF010 as host, and cerulenin-resistant strains were selected in a YPGal plate medium (1% yeast extract, 2% polypeptone, 2% galactose, 2% agar) containing 1.0 mg/L cerulenin.

The constitutive expression was confirmed by RT-PCR Total RNA was extracted by RNeasy Mini Kit (Qiagen) in accordance with the manual attached to the Kit. As ScSUL2 specific primers, ScSUL2_F2 (SEQ ID NO: 7) and ScSUL2_R3 (SEQ ID NO: 8) were used. As non_ScSUL2 specific primers, non-ScSUL2_F2 (SEQ ID NO: 9) and non-ScSUL2_R3 (SEQ ID NO: 10) were used. As internal standard, PDA1_for51 (SEQ ID NO: 11) and PDAI_730rv (SEQ ID NO: 12) specific to pyruvic acid dehydrogenase gene PDA1, were used. The PCR products were developed by agarose electrophoresis, and stained with an ethidium bromide solution. The signal value of each of the SUL2 genes was standardized with reference to the signal value of the PDA I gene. The strains having showed twice or more expression level of the parent TF010, were designated as ScSUL2- or non-ScSUL2-highly expressed strains. Two strains were selected for each of ScSUL2 and non-ScSUL2 genes.

### Example 3: Analysis of Amount of Sulfite Produced during Beer Brewing Testing

The parent strain TF010, and ScSUL2-highly expressed strains (two strains) and non-ScSUL2-highly expressed strains (two strains) obtained in Example 2, were used to carry out beer brewing testing under the following conditions.

| | |
|---|---|
| Wort extract concentration | 13% |
| Wort content | 800 mL |
| Wort dissolved oxygen concentration | about 8 ppm |
| Fermentation temperature | 15°C - stable |
| Yeast input | 5 g wet yeast fungal body/800 mL Wort extract |

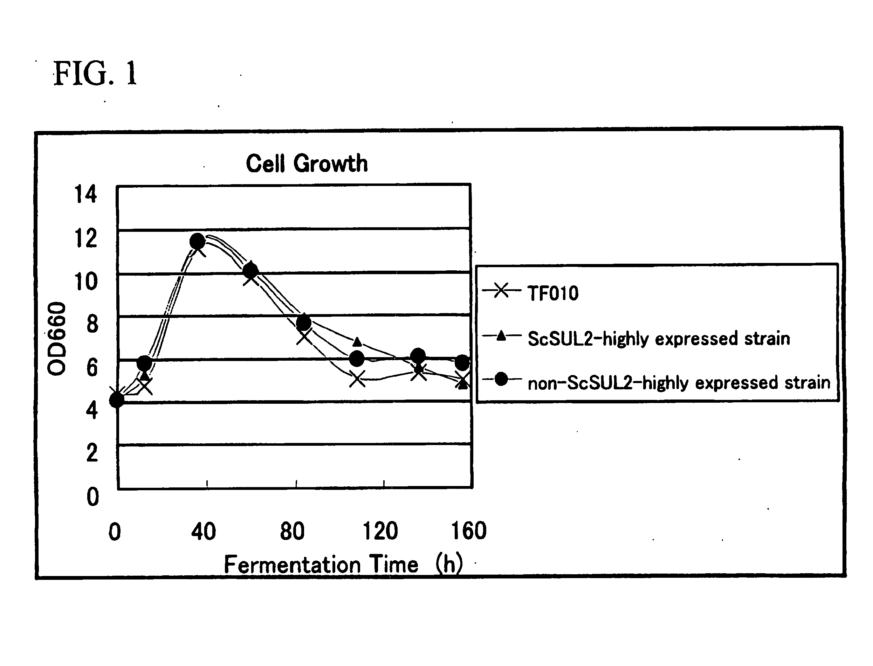

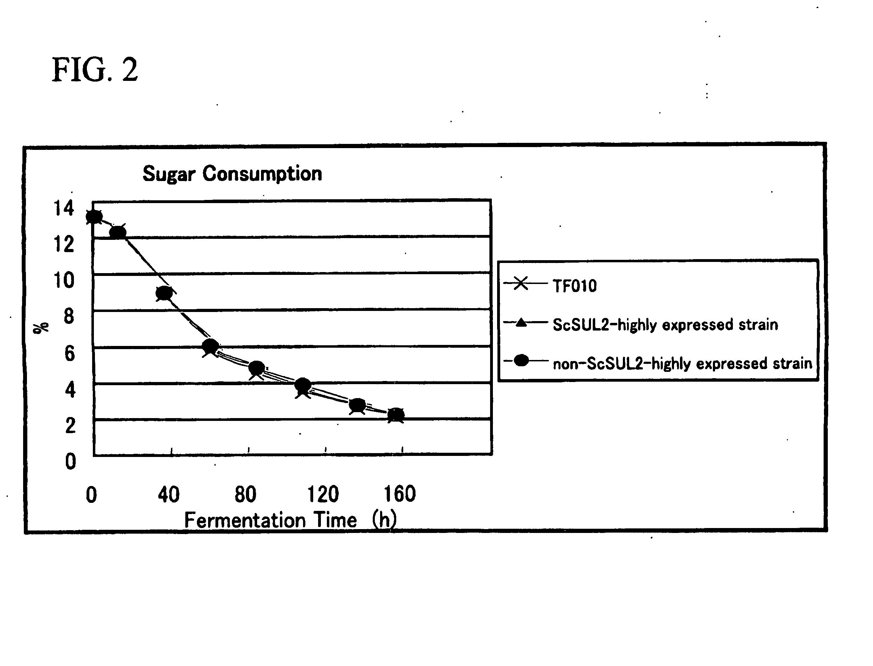

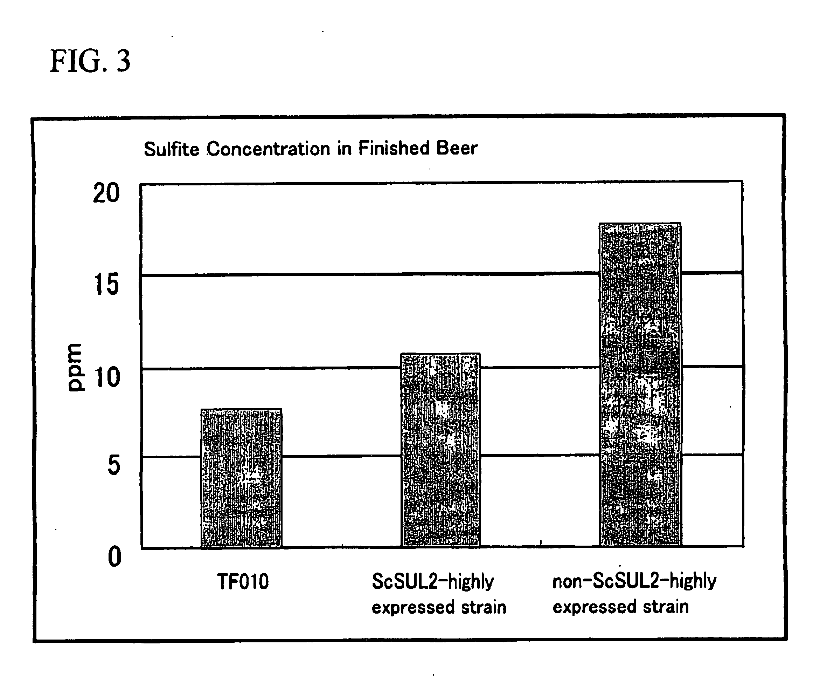

The fermentation broth was sampled with time to observe the cell growth (shown in Figure 1) and sugar consumption (shown in Figure 2) with time. Quantification of the sulfite content upon completion of fermentation was carried out by collecting sulfite in hydrogen peroxide solution by distillation under acidic condition, and titration with alkali (Revised BCOJ Beer Analysis Method by the Brewing Society of Japan). The results are shown in Figure 3. The results are shown in average of the data obtained from the two strains.

With respect to the amount of sulfite produced upon completion of fermentation, while the parent strain TF010 produced 8 ppm, the ScSUL2-highly expressed strains produced 10 ppm and the non-ScSUL2-highly expressed strains produced 18 ppm. Thus, it was found that the amount of sulfite produced can be remarkably increased by high expression of the non-ScSUL2. In these cases, differences in the growth rates and the extract consumption rates were little between the parent strain and the constitutively expressed strains.

As can be appreciated from the above results, by constitutively expressing sulfate ion transporter unique to a lager brewing yeast as described herein in the yeast with enhanced sulfite-producing capability, it became possible to specifically increase production amount of sulfite functioning as anti-oxidant for alcoholic beverages such as beer without altering, the fermentation procedure or time. Thus, alcoholic beverages with enhanced flavor and long shelf life (with good quality), can be produced.

### Example 4: Beer Brewing Testing using wort containing low sulfur source

*Saccharomyces pastorianus* Weihenstephan 34/70 strain is transformed with the high expression vector prepared in Example 2 to obtain, Sc and non-ScSUL2 (sole) highly expressed strains, respectively. Then, a wort containing 24% of malt ratio is prepared as a wort containing low sulfur source. Subsequently, using paraent and the highly expressed strains obtained, under the following conditions.beer brewing testing is carried out

| | |
|---|---|
| Wort extract concentration | 13% |
| Wort content | 2L |
| Wort dissolved oxygen concentration | about 8ppm |
| Fermentation temperature | 15°C constantly |
| Yeast input | 10.5 g of wet yeast cells/2 L of wort |

The fermentation broth is sampled with time to observe the cell growth (OD660) and the sugar consumption with time.

### Example 5: Disruption of SUL2 gene

According to the publication (Goldstein et al., yeast 15 1541 (1999)), PCR using a plasmid including a drug-resistant marker (pFA6a (*G418*) or pAG25 (*natl*)) as a template is conducted to prepare a fragment for SUL2 gene disruption.

With the fragment for gene disruption prepared, W34/70 strain or spore cloning strain (W34/70-2) is transformed. The transformation is performed in accordance with the method described in Patent Kokai H07-303475. The concentrations of the drugs for selection are 300 mg/L for geneticin and 50 mg/L of nourseothricin, respectively.

### Example 6: Analysis of amounts of sulfur-containing compound produced upon beer brewing testing

Using parent strain and the gene-disrupted strain obtained in Example 5, under the following conditions, beer brewing testing is carried out.

| | |
|---|---|
| Wort extract concentration | 13% |
| Wort content | 2L |
| Wort dissolved oxygen concentration | about 8ppm |
| Fermentation temperature | 15°C constantly |
| Yeast input | 10.5 g of wet yeast cells/2 L of wort |

The fermentation broth is sampled with time to observe the cell growth (OD660) and the sugar consumption with time. Analysis of sulfur-containing compounds in broth is performed by employing head-space gas chromatography.

### Industrial Applicability

According to the method for producing alcoholic beverages of the present invention, because of increase in content of sulfite having anti-oxidative action in a product, alcoholic beverages with enhanced flavor and long shelf life (with good quality), can be produced Also, since the yeast of the present invention can efficiently uptake a sulphate ion as a sulfur source, desirable alcoholic fermentation can be performed by using raw materials with low contents of sulfur-containing amino acid, e.g., sparkling liquor (*happoushu*) wort. Moreover, by suppressing an expression of said gene in yeast wherein sulfur-containing compounds as an off-flavor are highly generated, an alcoholic beverage having desirable flavor can be produced.

This application claims benefit of Japanese Patent Application No. 2005-177821 field June 17, 2005.

## Claims

1. A polynucleotide selected from the group consisting of:
(a) a polynucleotide comprising a polynucleotide consisting of the nucleotide' sequence of SEQ ID NO:1;
(b) a polynucleotide comprising a polynucleotide encoding a protein consisting of the amino acid sequence of SEQ ID NO:2;
(c) a polynucleotide encoding an amino acid sequence of SEQ ID NO: 2 wherein 1 to 50 amino acids thereof is deleted, substituted, inserted, and/or added, and wherein said protein has sulfate ion transporter activity; and
(d) a polynucleotide encoding a protein having 94% or higher identity with the amino add sequence of SEQ ID NO: 2, and having sulfate ion transporter activity.

2. the polynucleotide of Claim 1 consisting of SEQ ID NO: 1.

3. the polynucleotide of Claim 1 encoding a protein of SEQ ID NO: 2.

4. The polynucleotide of Claim 1, wherein the polynucleotide is DNA.

5. A polynucleotide selected from the following polynucleotides (j) to (m):
(j) a polynuycleotide encoding RNA of a nucleotide sequence complementary to a transcript of the polynucleotide (DNA) according to Claim 4;
(k) a polynucleotide encoding RNA that represses the expression of the polynucleotide (DNA) according to Claim 4 through RNAi effect:
(I) a polynucleotide encoding RNA having an activity of specifically cleaving a transcript of the polynucleotide (DNA) according to Claim 4; and
(m) a polynucleotide encoding RNA that represses expression of the polynucleotide (DNA) according to Claim 4 through co-supression effect.

6. A protein encoded by the polynucleotide of any of Claims 1 to 4.

7. A vector comprising the polynucleotide of any of Claims 1 to 4.

8. A vector comprising the polynucleotide of Claim 5.

9. A yeast comprising the vector of Claim 7 or 8.

10. The yeast of Claim 9, wherein a sulfite producing ability is enhanced by introducing the vector of claim 7.

11. A yeast, wherein an expression of the polynucleotide (DNA) of Claim 4 is suppressed by introducing the vector of Claim 8, or by disrupting a gene related to the polynucleotide (DNA) of Claim 4.

12. A yeast expressing the protein of Claim 6, wherein said protein expression increases sulfite production by said yeast.

13. A method for producing an alcoholic beverage comprising culturing the yeast of any one of Claim 9 to 12 under conditions for producing an alcoholic beverage.

14. The method for producing an alcoholic beverage of Claim 13, wherein the brewed alcoholic beverage is a malt beverage.

15. The method for producing an alcoholic beverage of Claim 13, wherein the brewed alcoholic beverage is wine.

16. A method for assessing a test yeast for its sulfite-producing capability, comprising using a primer or a probe designed based on a nucleotide sequence of a sulfate ion transporter gene having the nucleotide sequence of SEQ ID NO:1.

17. A method for assessing a test yeast for its sulfite-producing capability, comprising:
culturing a test yeast; and measuring an expression level of a sulfate ion transporter gene having the nucleotide sequence of SEQ ID NO:1.

18. A method for selecting a yeast, comprising: culturing test yeasts; quantifying the protein according to Claim 6 or measuring an expression level of a sulfate ion transporter gene having the nucleotide sequence of SEQ ID NO: 1; and selecting a test yeast having said protein amount or said gene expression level according to a target capability of producing sulfite.

19. The method for selecting a yeast according to Claim 18, comprising: culturing a reference yeast and test yeasts; measuring an expression level of a sulfate ion transporter gene having the nucleotide sequence of SEQ ID NO: 1 in each yeast; and selecting a test yeast having the gene expressed higher or lower than that in the reference yeast.

20. The method for selecting a yeast according to Claim 18, comprising: culturing a reference yeast and test yeasts; quantifying the protein according to Claim 6 in each yeast; and selecting a test yeast having said protein for a larger or smaller amount than that in the reference yeast.

21. A method for producing an alcoholic beverage comprising: conducting fermentation for producing an alcoholic beverage using the yeast according to any one of Claims 9 to 12 or a yeast selected by the method according to any one of Claims 18 to 20; and adjusting the production amount of sulfite.

## Patentansprüche

1. Polynucleotid, ausgewählt aus der Gruppe bestehend aus:
(a) einem Polynucleotid, umfassend ein Polynucleotid, das aus der Nucleotidsequenz von SEQ ID NO: 1 besteht;
(b) einem Polynucleotid, umfassend ein Polynucleotid, das ein Protein codiert, das aus der Aminosäuresequenz von SEQ ID NO: 2 besteht;
(c) einem Polynucleotid, das eine Aminosäuresequenz der SEQ ID NO: 2 codiert, wobei 1 bis 50 Aminosäuren davon deletiert, ausgetauscht, eingeführt und/oder hinzugefügt sind und wobei das Protein Sulfationtransporteraktivität aufweist; und
(d) einem Polynucleotid, das ein Protein mit 94% oder höherer Identität mit der Aminosäuresequenz von SEQ ID NO: 2 und mit Sulfationtransporteraktivität codiert.

2. Polynucleotid gemäß Anspruch 1, das aus SEQ ID NO: 1 besteht.

3. Polynucleotid gemäß Anspruch 1, das ein Protein der SEQ ID NO: 2 codiert.

4. Polynucleotid gemäß Anspruch 1, wobei das Polynucleotid DNA ist.

5. Polynucleotid ausgewählt aus den folgenden Polynucleotiden (j) bis (m):
(j) einem Polynucleotid, das RNA mit einer Nucleotidsequenz codiert, die komplementär zu einem Transkript des Polynucleotids (DNA) gemäß Anspruch 4 ist;
(k) einem Polynucleotid, das RNA codiert, die die Expression des Polynucleotids (DNA) gemäß Anspruch 4 durch einen RNAi-Effekt unterdrückt;
(l) einem Polynucleotid, das RNA codiert, die eine Aktivität zum spezifischen Schneiden eines Transkripts des Polynucleotids (DNA) gemäß Anspruch 4 aufweist; und
(m) einem Polynucleotid, das RNA codiert, die die Expression des Polynucleotids (DNA) gemäß Anspruch 4 durch Co-Suppressionseffekt unterdrückt.

6. Protein, das durch ein Polynucleotid gemäß einem der Ansprüche 1 bis 4 codiert wird.

7. Vektor, der das Polynucleotid gemäß einem der Ansprüche 1 bis 4 umfasst.

8. Vektor, der das Polynucleotid gemäß Anspruch 5 umfasst.

9. Hefe, die den Vektor gemäß Anspruch 7 oder 8 umfasst.

10. Hefe gemäß Anspruch 9, wobei eine Sulfit-produzierende Fähigkeit durch das Einführen des Vektors gemäß Anspruch 7 erhöht ist.

11. Hefe, in der eine Expression des Polynucleotids (DNA) gemäß Anspruch 4 durch das Einführen des Vektors gemäß Anspruch 8 oder durch Stören eines Gens, das mit dem Polynucleotid (DNA) gemäß Anspuch 4 in Zusammenhang steht, unterdrückt ist.

12. Hefe, die das Protein gemäß Anspruch 6 exprimiert, wobei die Proteinexpression die Sulfitproduktion durch die Hefe erhöht.

13. Verfahren zum Herstellen eines alkoholischen Getränks, das das Züchten der Hefe gemäß einem der Ansprüche 9 bis 12 unter Bedingungen zum Herstellen eines alkoholischen Getränks umfasst.

14. Verfahren zum Herstellen eines alkoholischen Getränks gemäß Anspruch 13, wobei das gebraute alkoholische Getränk ein Malzgetränk ist.

15. Verfahren zum Herstellen eines alkoholischen Getränks gemäß Anspruch 13, wobei das gebraute alkoholische Getränk Wein ist.

16. Verfahren zum Beurteilen einer Testhefe im Hinblick auf deren Sulfit-bildende Fähigkeit, das das Verwenden eines Primers oder einer Sonde umfasst, die auf der Basis einer Nucleotidsequenz eines Sulfationtranportergens mit der Nucleotidsequenz von SEQ ID NO: 1 entworfen wurden.

17. Verfahren zum Beurteilen einer Testhefe im Hinblick auf deren Sulfit-bildende Fähigkeit, das umfasst: das Züchten einer Testhefe; und das Messen eines Expressionsspiegels eines Sulftationtransportergens mit der Nucleotidsequenz von SEQ ID NO: 1.

18. Verfahren zum Auswählen einer Hefe, das umfasst: das Züchten von Testhefen; das Quantifizieren des Proteins gemäß Anspruch 6 oder das Messen eines Expressionsspiegels eines Sulfationtransportergens mit der Nucleotidsequenz von SEQ ID NO: 1; und das Auswählen einer Testhefe, die die Proteinmenge oder den Gen-Expressionsspiegel gemäß einer Zielfähigkeit zur Sulfitproduktion hat.

19. Verfahren zum Auswählen einer Hefe gemäß Anspruch 18, das umfasst: das Züchten einer Referenzhefe und von Testhefen; das Messen eines Expressionsspiegel eines Sulfationtransportergens mit der Nucleotidsequenz von SEQ ID NO: 1 in jeder Hefe; und das Auswählen einer Testhefe, bei der das Gen höher oder niedriger als das in der Referenzhefe exprimiert wird.

20. Verfahren zum Auswählen einer Hefe gemäß Anspruch 18, das umfasst: das Züchten einer Referenzhefe und von Testhefen; das Quantifizieren des Proteins gemäß Anspruch 6 in jeder Hefe; und das Auswählen einer Testhefe, die das Protein in einer größeren oder kleineren Menge als das in der Referenzhefe aufweist.

21. Verfahren zum Herstellen eines alkoholischen Getränks, das umfasst: das Durchführen einer Fermentation zur Herstellung eines alkoholischen Getränks unter Verwendung der Hefe gemäß einem der Ansprüche 9 bis 12 oder einer Hefe, die durch das Verfahren gemäß einem der Ansprüche 18 bis 20 ausgewählt wurde; und das Angleichen der Produktionsmenge an Sulfit.

## Revendications

1. Polynucléotide choisi dans le groupe comprenant :
(a) un polynucléotide comprenant un polynucléotide constitué de la séquence nucléotidique de SEQ ID NO : 1 ;
(b) un polynucléotide comprenant un polynucléotide codant pour une protéine constituée de la séquence d'acides aminés de SEQ ID NO : 2 ;
(c) un polynucléotide codant pour une séquence d'acides aminés de SEQ ID NO : 2 dans laquelle 1 à 50 acides aminés de celles-ci sont délétés, substitués, insérés et/ou ajoutés, et où ladite protéine possède une activité de transporteur des ions sulfate ; et
(d) un polynucléotide codant pour une protéine présentant une identité de 94 % ou supérieure avec la séquence d'acides aminés de SEQ ID NO : 2, et possédant une activité de transporteur des ions sulfate.

2. Polynucléotide selon la revendication 1, constitué de SEQ ID NO : 1.

3. Polynucléotide selon la revendication 1, codant pour une protéine de SEQ ID NO : 2.

4. Polynucléotide selon la revendication 1, où le polynucléotide est de l'ADN.

5. Polynucléotide choisi parmi les polynucléotides (j) à (m) suivants :
(j) un polynucléotide codant pour l'ARN d'une séquence nucléotidique complémentaire d'un transcrit du polynucléotide (ADN) selon la revendication 4 ;
(k) un polynucléotide codant pour l'ARN qui réprime l'expression du polynucléotide (ADN) selon la revendication 4 par l'intermédiaire d'un effet ARNi ;
(l) un polynucléotide codant pour l'ARN possédant une activité de clivage spécifique d'un transcrit du polynucléotide (ADN) selon la revendication 4 ; et
(m) un polynucléotide codant pour l'ARN qui réprime l'expression du polynucléotide (ADN) selon la revendication 4 par l'intermédiaire d'un effet de cosuppression.

6. Protéine codée par le polynucléotide selon l'une quelconque des revendications 1 à 4.

7. Vecteur comprenant le polynucléotide selon l'une quelconque des revendications 1 à 4.

8. Vecteur comprenant le polynucléotide selon la revendication 5.

9. Levure comprenant le vecteur selon la revendication 7 ou 8.

10. Levure selon la revendication 9, dans laquelle la capacité à produire des sulfites est amplifiée par l'introduction du vecteur selon la revendication 7.

11. Levure dans laquelle une expression du polynucléotide (ADN) selon la revendication 4 est réprimée par l'introduction du vecteur selon la revendication 8, ou par la rupture d'un gène associé au polynucléotide (ADN) selon la revendication 4.

12. Levure exprimant la protéine selon la revendication 6, dans laquelle l'expression de ladite protéine augmente la production de sulfites par ladite levure.

13. Procédé de production d'une boisson alcoolisée comprenant la culture de la levure selon l'une quelconque des revendications 9 à 12 dans des conditions de production d'une boisson alcoolisée.

14. Procédé de production d'une boisson alcoolisée selon la revendication 13, dans lequel la boisson alcoolisée brassée est une boisson à base de malt.

15. Procédé de production d'une boisson alcoolisée selon la revendication 13, dans lequel la boisson alcoolisée brassée est du vin.

16. Procédé permettant d'estimer une levure à tester pour sa capacité à produire des sulfites, comprenant l'utilisation d'une amorce ou d'une sonde conçue d'après une séquence nucléotidique d'un gène de transporteur des ions sulfate ayant la séquence nucléotidique de SEQ ID NO : 1.

17. Procédé permettant d'estimer une levure à tester pour sa capacité à produire des sulfites, comprenant : la culture d'une levure à tester ; et la mesure d'un taux d'expression d'un gène de transporteur des ions sulfate ayant la séquence nucléotidique de SEQ ID NO : 1.

18. Procédé permettant de sélectionner une levure, comprenant : la culture de levures à tester ; la quantification de la protéine selon la revendication 6 ou la mesure d'un taux d'expression d'un gène de transporteur des ions sulfate ayant la séquence nucléotidique de SEQ ID NO : 1 ; et la sélection d'une levure à tester présentant ladite quantité de protéine ou ledit taux d'expression du gène selon une capacité cible de production de sulfites.

19. Procédé permettant de sélectionner une levure selon la revendication 18, comprenant : la culture d'une levure de référence et de levures à tester ; la mesure d'un taux d'expression d'un gène de transporteur des ions sulfate ayant la séquence nucléotidique de SEQ ID NO : 1 dans chaque levure ; et la sélection d'une levure à tester présentant le gène exprimé supérieur ou inférieur à celui dans la levure de référence.

20. Procédé permettant de sélectionner une levure selon la revendication 18, comprenant : la culture d'une levure de référence et de levures à tester ; la quantification de la protéine selon la revendication 6 dans chaque levure ; et la sélection d'une levure à tester présentant ladite protéine pour une quantité supérieure ou inférieure à celle dans la levure de référence.

21. Procédé de production d'une boisson alcoolisée comprenant: la conduite d'une fermentation pour produire une boisson alcoolisée en utilisant la levure selon l'une quelconque des revendications 9 à 12 ou une levure sélectionnée par le procédé selon l'une quelconque des revendications 18 à 20 ; et l'ajustement de la quantité de production de sulfites.


### SEQUENCE LISTING

<110> Suntory Limited
<120> Sulfate ion tranporter gene and its use
<130> PCT06-0044
<150> JP2005-177821
   <151> 2005-06-17
<160> 12
<170> Patent In version 3.1
<210> 1
   <211> 2685
   <212> DNA
   <213> yeast
<400> 1
   atgccaaggg aaacacattc gagtatcgaa gaagaggtgg aaatccccga ttttcaagat     60
   agcgcaacag ttcctgatct tgatgactta gagctggagt acgaccaata caaaaataat    120
   gaaaacaatg gtcccttcaa cgataatgat ctggaaagca attccgttgc caaccacaac    180
   acggtgagca attcgaaggg cattaatggc tcgaaggtcg agtattttaa cgcttcggat    240
   atctcgttgt atgacaattc tgtttcgcag ttcgaagaat ctactgtgtc gttcaaagat    300
   tactacgacc atagtatacg ttctcatctt actttgaaag gtgctagcga ttatcttaaa    360
   agcgtctttc caatcattaa ctggttgcca cactataacg ttagttggtt tattgcagat    420
   ctgatcgcgg gtatcactat cggctgtgtt cttgtgccac aatccatgtc gtatgcgcaa    480
   gtcgccacat tgcctgcaca gtacgggctg tactcttcat ttattggtgc ctatgcctat    540
   gcgttttttg caacctccaa ggatgtttgt attggacccg tcgccgtcat gtccttacag    600
   accgccaaag ttattgcaga tgtcacatcc agatacccag aaggtgacac taccattacg    660
   ggtcccatca tcgccacgac attggcgtta ttgtgtggga ttatttctgc tgctgttggt    720
   ttcttacgtt tggggttcct tgtcgaattc atttctttaa atgctgtcgc tgggtttatg    780
   accgggtctg cctttaacat tttgtggggc caagtgccag ctcttatggg gtacaacaaa    840
   ttggtcaata caagagtcgc cacttataag atcgtgatag actctttgaa acatttaccg    900
   gacactacac tggatgctgt gtttggactt atcccacttt tcattcttta cacttggaaa 960
   tggtggtgtg gtacatatgg tccaaagttg aatgacagat tcaattctaa aaggccaaga 1020
   ttacacaaga ttgtcaaatg gacatatttc tacgcacagg cttctagaaa tggtattatt 1080
   atcattgtat tcacttgtat tgcttgggcc atcacaagag gcaaatctgc agctgacaga 1140
   ccaatcagtg tcctgggatc cgttccatcc ggtttgaaag aggtcggtgt cttccatgtt 1200
   ccacccggac tgatgtctaa attgggtcca aatctgcctg cttctattat cgtcttgcta 1260
   ttagagcata ttgctatttc caaatcattc ggtagagtta acgattacaa agttgtccct 1320
   gatcaagaat taattgctat tggtgtttcg aacttattag gtaccttctt taatgcctat 1380
   ccagccactg gttccttctc gagatctgct ttgaaggcta aatgtaatgt tcgtacccca 1440
   ctatctggtt tattttcagg ttcttgtgtg cttttagcat tatactgttt aactggcgca 1500
   ttcttttaca ttccaaaggc cacattatcg gctgttatca tccacgcagt gtctgatctt 1560
   ctggcctcct accaaacaac ctggaatttc tggaaaatga acccactgga cttcgtctgt 1620
   ttcattgtta cagtcttgat cacggtcttt tcctccattg aaaatggtat ttatttcgct 1680
   atgtgctggt catgtgcaat cttcatactt aaagtggcat tcccagctgg taaattcttg 1740
   ggtcgtgtcg aaatcgccga agttactgat gcttatgtca gagctgattc ggacagtgtt 1800
   agcttcacat ctgaaaacaa caatcgtatt ttaacattgg aagaagatac tactgatggt 1860
   gaaaaggagg ggcgtaccgg ttatataatt aactcctcta aaaaactaga cacaaaagtc 1920
   caaacgaagg gtttcgaatc cccatcccct tcaattggcc aaccaagaat aaagtatcac 1980
   accaaatggc taccattcga ccacaaatac acaagagaat taaacccgga tgtccagatt 2040
   ctcccacctc cagatggtgt cctcgtttac aggctatccg atagttacac gtatatcaat 2100
   tgttcaagac actatgatat tataatagac cgagtcaaga aggtaacgag acgtggtcaa 2160
   ctaattcgcc acaggaaaaa atccgaccgc ccttggaacg atcctggtcc atgggaacca 2220
   cctactttct ttaagaattt gaaattctgg aagaaaaagg aaacggatat cgagcctgcc 2280
   gaaaatgctg ccaacaaccc aactgatgct gaccgagatg atagaccatt actaaaaatc 2340
   gtgtgtttgg atttctcgca agtggcgcaa acagatgcca ctgctcttca gtcactggtt 2400
   gacctaagga aagtcatcaa ccaatatgct gatagacaag ttgaattcca tttcgttggt 2460
   atcatttcat catgggtgaa gagaggtttg attagtagag gttttggtac tctaaacgaa 2520
   gaatatagtg atgaatccat tgttgccggt catacaagtt atcacgtcgc aagagtacct 2580
   caaaacaaag aaaccccagg caaatatagc gtttacactg tttcaggtac aaacttacct 2640
   ttcttccata tagatattcc agactttgct aaatgggaca tttag 2685
<210> 2
   <211> 894
   <212> PRT
   <213> yeast
<400> 2
   Met Pro Arg Glu Thr His Ser Ser Ile Glu Glu Glu Val Glu Ile Pro
   1 5 10 15
   Asp Phe Gln Asp Ser Ala Thr Val Pro Asp Leu Asp Asp Leu Glu Leu
   20 25 30
   Glu Tyr Asp Gln Tyr Lys Asn Asn Glu Asn Asn Gly Pro Phe Asn Asp
   35 40 45
   Asn Asp Leu Glu Ser Asn Ser Val Ala Asn His Asn Thr Val Ser Asn
   50 55 60
   Ser Lys Gly Ile Asn Gly Ser Lys Val Glu Tyr Phe Asn Ala Ser Asp
   65 70 75 80
   Ile Ser Leu Tyr Asp Asn Ser Val Ser Gln Phe Glu Glu Ser Thr Val
   85 90 95
   Ser Phe Lys Asp Tyr Tyr Asp His Ser Ile Arg Ser His Leu Thr Leu
   100 105 110
   Lys Gly Ala Ser Asp Tyr Leu Lys Ser Val Phe Pro Ile Ile Asn Trp
   115 120 125
   Leu Pro His Tyr Asn Val Ser Trp Phe Ile Ala Asp Leu Ile Ala Gly
   130 135 140
   Ile Thr Ile Gly Cys Val Leu Val Pro Gln Ser Met Ser Tyr Ala Gln
   145 150 155 160
   Val Ala Thr Leu Pro Ala Gln Tyr Gly Leu Tyr Ser Ser Phe Ile Gly
   165 170 175
   Ala Tyr Ala Tyr Ala Phe Phe Ala Thr Ser Lys Asp Val Cys Ile Gly
   180 185 190
   Pro Val Ala Val Met Ser Leu Gln Thr Ala Lys Val Ile Ala Asp Val
   195 200 205
   Thr Ser Arg Tyr Pro Glu Gly Asp Thr Thr Ile Thr Gly Pro Ile Ile
   210 215 220
   Ala Thr Thr Leu Ala Leu Leu Cys Gly Ile Ile Ser Ala Ala Val Gly
   225 230 235 240
   Phe Leu Arg Leu Gly Phe Leu Val Glu Phe Ile Ser Leu Asn Ala Val
   245 250 255
   Ala Gly Phe Met Thr Gly Ser Ala Phe Asn Ile Leu Trp Gly Gln Val
   260 265 270
   Pro Ala Leu Met Gly Tyr Asn Lys Leu Val Asn Thr Arg Val Ala Thr
   275 280 285
   Tyr Lys Ile Val Ile Asp Ser Leu Lys His Leu Pro Asp Thr Thr Leu
   290 295 300
   Asp Ala Val Phe Gly Leu Ile Pro Leu Phe Ile Leu Tyr Thr Trp Lys
   305 310 315 320
   Trp Trp Cys Gly Thr Tyr Gly Pro Lys Leu Asn Asp Arg Phe Asn Ser
   325 330 335
   Lys Arg Pro Arg Leu His Lys Ile Val Lys Trp Thr Tyr Phe Tyr Ala
   340 345 350
   Gln Ala Ser Arg Asn Gly Ile Ile Ile Ile Val Phe Thr Cys Ile Ala
   355 360 365
   Trp Ala Ile Thr Arg Gly Lys Ser Ala Ala Asp Arg Pro Ile Ser Val
   370 375 380
   Leu Gly Ser Val Pro Ser Gly Leu Lys Glu Val Gly Val Phe His Val
   385 390 395 400
   Pro Pro Gly Leu Met Ser Lys Leu Gly Pro Asn Leu Pro Ala Ser Ile
   405 410 415
   Ile Val Leu Leu Leu Glu His Ile Ala Ile Ser Lys Ser Phe Gly Arg
   420 425 430
   Val Asn Asp Tyr Lys Val Val Pro Asp Gln Glu Leu Ile Ala Ile Gly
   435 440 445
   Val Ser Asn Leu Leu Gly Thr Phe Phe Asn Ala Tyr Pro Ala Thr Gly
   450 455 460
   Ser Phe Ser Arg Ser Ala Leu Lys Ala Lys Cys Asn Val Arg Thr Pro
   465 470 475 480
   Leu Ser Gly Leu Phe Ser Gly Ser Cys Val Leu Leu Ala Leu Tyr Cys
   485 490 495
   Leu Thr Gly Ala Phe Phe Tyr Ile Pro Lys Ala Thr Leu Ser Ala Val
   500 505 510
   Ile Ile His Ala Val Ser Asp Leu Leu Ala Ser Tyr Gln Thr Thr Trp
   515 520 525
   Asn Phe Trp Lys Met Asn Pro Leu Asp Phe Val Cys Phe Ile Val Thr
   530 535 540
   Val Leu Ile Thr Val Phe Ser Ser Ile Glu Asn Gly Ile Tyr Phe Ala
   545 550 555 560
   Met Cys Trp Ser Cys Ala Ile Phe Ile Leu Lys Val Ala Phe Pro Ala
   565 570 575
   Gly Lys Phe Leu Gly Arg Val Glu Ile Ala Glu Val Thr Asp Ala Tyr
   580 585 590
   Val Arg Ala Asp Ser Asp Ser Val Ser Phe Thr Ser Glu Asn Asn Asn
   595 600 605
   Arg Ile Leu Thr Leu Glu Glu Asp Thr Thr Asp Gly Glu Lys Glu Gly
   610 615 620
   Arg Thr Gly Tyr Ile Ile Asn Ser Ser Lys Lys Leu Asp Thr Lys Val
   625 630 635 640
   Gln Thr Lys Gly Phe Glu Ser Pro Ser Pro Ser Ile Gly Gln Pro Arg
   645 650 655
   Ile Lys Tyr His Thr Lys Trp Leu Pro Phe Asp His Lys Tyr Thr Arg
   660 665 670
   Glu Leu Asn Pro Asp Val Gln Ile Leu Pro Pro Pro Asp Gly Val Leu
   675 680 685
   Val Tyr Arg Leu Ser Asp Ser Tyr Thr Tyr Ile Asn Cys Ser Arg His
   690 695 700
   Tyr Asp Ile Ile Ile Asp Arg Val Lys Lys Val Thr Arg Arg Gly Gln
   705 710 715 720
   Leu Ile Arg His Arg Lys Lys Ser Asp Arg Pro Trp Asn Asp Pro Gly
   725 730 735
   Pro Trp Glu Pro Pro Thr Phe Phe Lys Asn Leu Lys Phe Trp Lys Lys
   740 745 750
   Lys Glu Thr Asp Ile Glu Pro Ala Glu Asn Ala Ala Asn Asn Pro Thr
   755 760 765
   Asp Ala Asp Arg Asp Asp Arg Pro Leu Leu Lys Ile Val Cys Leu Asp
   770 775 780
   Phe Ser Gln Val Ala Gln Thr Asp Ala Thr Ala Leu Gln Ser Leu Val
   785 790 795 800
   Asp Leu Arg Lys Val Ile Asn Gln Tyr Ala Asp Arg Gln Val Glu Phe
   805 810 815
   His Phe Val Gly Ile Ile Ser Ser Trp Val Lys Arg Gly Leu Ile Ser
   820 825 830
   Arg Gly Phe Gly Thr Leu Asn Glu Glu Tyr Ser Asp Glu Ser Ile Val
   835 840 845
   Ala Gly His Thr Ser Tyr His Val Ala Arg Val Pro Gln Asn Lys Glu
   850 855 860
   Thr Pro Gly Lys Tyr Ser Val Tyr Thr Val Ser Gly Thr Asn Leu Pro
   865 870 875 880
   Phe Phe His Ile Asp Ile Pro Asp Phe Ala Lys Trp Asp Ile
   885 890
<210> 3
   <211> 21
   <212> DNA
   <213> Artificial
<220>
   <223> primer
<400> 3
   aagatatgtc cagggaaggt t 21
<210> 4
   <211> 21
   <212> DNA
   <213> Artificial
<220>
   <223> primer
<400> 4.
   ttgcatgtcc attgtaaatg c 21
<210> 5
   <211> 21
   <212> DNA
   <213> Artificial
<220>
   <223> primer
<400> 5
   tagttatgcc aagggaaaca c 21
<210> 6
   <211> 24
   <212> DNA
   <213> Artificial
<220>
   <223> primer
<400> 6
   gatttataaa tatgttgtaa ggtg 24
<210> 7
   <211> 20
   <212> DNA
   <213> Artificial
<220>
   <223> primer
<400> 7
   caaagtcatt gcggatgtca 20
<210> 8
   <211> 21
   <212> DNA
   <213> Artificial
<220>
   <223> primer
<400> 8
   gacgtaagca tcagtaactt c 21
<210> 9
   <211> 21
   <212> DNA
   <213> Artificial
<220>
   <223> primer
<400> 9
   caaagttatt gcagatgtca c 21
<210> 10
   <211> 22
   <212> DNA
   <213> Artificial
<220>
   <223> primer
<400> 10
   gacataagca tcagtaactt cg 22
<210> 11
   <211> 18
   <212> DNA
   <213> Artificial
<220>
   <223> primer
<400> 11
   ggaaaccaca tttgtgcc 18
<210> 12
<211> 18
   <212> DNA
   <213> Artificial
<220>
   <223> primer
<400> 12
   gattagaggc accatcac 18